# EUROPEAN PATENT SPECIFICATION

(11) **EP 0 721 448 B1**
(45) Date of publication and mention of the grant of the patent: **11.03.1998**
(21) Application number: 94927719.8
(22) Date of filing: 26.09.1994
(51) Int. Cl.: C07C 255/43, C07C 253/34

(54) **CHIRAL NITRILES, THEIR PREPARATION AND THEIR USE FOR THE MANUFACTURE OF VERAPAMIL AND ANALOGUES**
CHIRALE NITRILE, IHRE HERSTELLUNG UND IHRE VERWENDUNG IN DER HERSTELLUNG VON VERAPAMIL UND DESSEN ANALOGEN
NITRILES CHIRAUX, LEUR PREPARATION ET LEUR UTILISATION DANS LA FABRICATION DE VERAPAMIL ET DE SES ANALOGUES

(30) Priority: 27.09.1993 GB 9319918
(43) Date of publication of application: 17.07.1996
(73) Proprietor: Chiroscience Limited, Cambridge CB4 4WE (GB)
(72) Inventor: MCCAGUE, Raymond, Cambridgeshire CB4 6EE (GB); WANG, Shouming, Cambridge CB4 3RN (GB)
(74) Representative: Hucker, Charlotte Jane
(86) International application number: GB9402091
(87) International publication number: WO9509150

(56) References cited:
- EP-A- 0 231 003
- EP-A- 0 434 093
- WO-A-93/16035
- DE-A- 2 059 923

## Description

This invention relates to chiral compounds. In particular, it relates to a process for the manufacture of verapamil and analogues thereof, more particularly the manufacture of the single enantiomers thereof, and intermediates in that process.

### Background of the Invention

Verapamil is presently in clinical use as the racemate and is used extensively for treatment of hypertension. The (S)-isomer has the majority of the calcium channel antagonist activity, see DE-A-2059923, whilst the (R)-isomer differs in having sodium channel and other cell-pump actions in addition to a higher bioavailability, with slower clearance rate. For the treatment of hypertension, the (S)-isomer may provide a safer treatment than the racemate, with an extended therapeutic window. The (R)-isomer may be of benefit for the treatment of multidrug resistance in cancer chemotherapy, see J.F. Eliason, H. Ramuz and F. Kaufmann, Int. J. Cancer (1990) 46: 113-117; in this case hypotensive action by admixture with (S)-isomer would be undesirable. The preferential use of one of the isomers for migraine treatment is also possible, see S.J. Peroutka, Ann. Neurol. (1988) 23: 500-504.

The preparation of the single enantiomers of verapamil is a difficult chemical problem. DE-A-3723654 discloses that the isomers have been separated by resolution with, for example, binaphthol bis(dihydrogen phosphate), but this would appear to be an expensive process. Similarly, the resolution of a racemic carboxylic acid precursor with brucine, as disclosed in DE-A-2059923, and the multi-step process thereafter, does not look attractive for bulk manufacture, nor does a lengthy synthesis from the enantiomers of propane-1,2-diol, see L.J. Theodore and W.L. Nelson, J. Org. Chem. (1987) 52: 1309-1315, or separation of the final product by chromatography, see JP-A-03027326.

### Summary of the Invention

According to a first aspect of the present invention, a process for preparing a substantially single enantiomer (R or S) of

R'-NH-(CH₂)₃-C(Ar)(CN)-R,

the process comprising reacting together

R-CH(Ar)-CN and X-(CH₂)₃-A

to give

X-(CH₂)₃-C(Ar)(CN)-R,

reacting that with R'NH₂ to give

R'-NH-(CH₂)₃-C(Ar)(CN)-R,

and resolving that into a substantially single enantiomer thereof, wherein Ar is aryl, R and R' are each independently C₁₋₂₀ alkyl, X is a halo atom and A is a leaving group and the resolution is carried out using a chiral acid.

According to a second aspect of the present invention, a process for preparing a substantially single enantiomer (R or S) of a compound of the formula

Ar'-(CH₂)₂-NR'-(CH₂)₃-C(Ar)(CN)-R

comprises carrying out the process according to the first aspect of the invention and reacting the product thereof with Ar'-(CH₂)₂-A', wherein Ar' is aryl and A' is a leaving group.

According to a third aspect of the present invention, a process for preparing a substantially single enantiomer (R or S) of verapamil comprises carrying out the process according to the second aspect of the invention, wherein Ar and Ar' are both 3,4-dimethoxyphenyl, R is isopropyl and R' is methyl.

Resolution of CH₃-NH-(CH₂)₃-C(Ar)(CN)-CH(CH₃)₂, Ar being 3,4-dimethoxyphenyl, is relatively easy compared with resolution of verapamil. For example, the novel resolution can directly achieve around 90%, or greater, enantiomeric excess, and after recrystallisation this may be increased to 99%, or greater, enantiomeric excess.

Apart from ease, a further benefit of resolution of CH₃-NH-(CH₂)₃-C(Ar)(CN)-CH(CH₃)₂, and its subsequent conversion to verapamil, over resolution of verapamil itself is that, given the inability to utilise both enantiomers of verapamil (which are not readily interconvertible), the amount of waste enantiomer will be reduced.

### Description of the Invention

The process of the invention is suitable for the manufacture of a wide range of chiral compounds having Ar, Ar' and R as defined above. Preferably, the process is for preparing verapamil and analogues thereof, such as emopamil (Ar = Ar'= phenyl, R = isopropyl, R' = methyl), gallopamil (Ar = 3,4,5-trimethoxyphenyl, Ar'= 3,4-dimethoxyphenyl, R = isopropyl, R' = methyl), devapamil (Ar = 3,4-dimethoxyphenyl, Ar'= 3-methoxyphenyl, R = isopropyl, R' = methyl), mepamil (Ar = 2-methylphenyl, Ar'= 3,4-dimethoxyphenyl, R = n-dodecyl, R' = methyl), ronipamil (Ar = Ar' = phenyl, R = n-dodecyl, R' = methyl), dagapamil (Ar = 3,4,5-trimethoxyphenyl, Ar'= 3-methoxyphenyl, R = n-dodecyl, R' = methyl), anapamil (Ar = 3-methoxyphenyl, Ar'= phenyl, R = n-dodecyl, R' = methyl), etc.

Preparation of R-CH(Ar)-CN follows similar chemistry to that for verapamil. Resolution is typically carried out with a derivative of tartaric acid, such as is produced by acylation at at least one of its hydroxyl groups, preferably di-o,o'-toluyltartaric acid, or with lithocholic acid or (R)-α-methoxy-2-(trifluoromethyl)phenylacetic acid. Conversion of the resolved compound to verapamil, or the respective analogue thereof, is by standard chemical steps.

The following Example illustrates the invention.

### Example

### a) Preparation of 5-Chloro-2-(3,4-dimethoxyphenyl)-2-isopropylpentanenitrile

2-(3,4-dimethoxyphenyl)-3-methylbutanenitrile (1.21 g, 5.52 mmol) was added to a suspension of sodium amide (0.59 g, 14.4 mmol) in toluene (12 ml). The mixture was then heated to reflux for 2 hours before the addition of 1-chloro-3-iodopropane (0.9 ml, 8.3 mmol). The mixture was cooled to room temperature over 80 minutes, and worked up by addition of water. The product was extracted with methyl-t-butyl ether (MTBE). After usual work-up, the product was purified on column chromatography eluting with (ethyl acetate: light petroleum (b.p. 40-60°C) = 1:2), to give 1.4g (82% yield) of the required product.

### b) Preparation of 2-(3,4-Dimethyoxyphenyl)-2-isopropyl-5-(methylamino)pentanenitrile

Into a sealed tube were placed the crude 5-chloro-2-(3,4-dimethoxyphenyl)-2-isopropylpentanenitrile from above (0.879 g, 2.97 mmol), in absolute ethanol (4 ml), K₂CO₃ (1.245 g, 8.92 mmol), NaI (0.05 g), and methylamine (1.85 ml, 33% in absolute ethanol). The mixture was then heated at 100°C for over 3 days, and the volatiles were removed on a rotary evaporator. The residue was extracted with diethyl ether. The combined organics were washed with water, then acidified with 2N hydrochloric acid. The organic phase was further extracted with 2N HCl (3 x 50 ml).

The combined acidic aqueous phase was then basified with 48% NaOH solution at 0°C. The product was then extracted out with diethyl ether. Usual work-up gave the desired product in essentially pure form (0.62g, 71%).

### c) Resolution of methylamino intermediate

The racemic methylamino intermediate (0.62 g, 2.1 mmol) was dissolved in methanol (2.5 ml) at room temperature, to which was added a solution of di-o,o'-toluyl-D-tartaric acid monohydrate (0.88 g, 2.1 mmol) in methanol (1.8 ml). The mixture was then cooled to 10-15 °C. MTBE (4 ml) was then added to the above mixture in four equal portions. After gentle stirring the solid salt began to appear slowly. The stirring was continued for about six hours, and then the mixture was left standing for 3 days. The solid was then filtered off, washed with MTBE (4 x 1 ml), and dried under vacuum, giving the diastereomeric salt (0.43 g, 29 %) with an ee of 93 %, S. (ee was determined by eluting the p-toluyl amide derived from the methylamino intermediate and p-toluyl chloride on to a chiral cel O.J.Column, 10%, iPA/heptane 90 %).

The solid salt was then dissolved in MeOH (3.4 ml) under gentle heating, and slowly cooled to room temperature. A trace amount of MTBE was added to the solution to facilitate recrystallisation. The salt obtained was then filtered, washed with MTBE (3.7 ml) and dried under vacuum to give the first crop (2.6 g, 61% yield, 99.96 % ee, S,(α)_{D} = (+)70.2 (c=1, abs. EtOH).

The combined liquors were left at room temperature to give second crop (0.6 g, 14%, 99 % ee, S). The subsequent liquor was then concentrated on rotavapour, and the third crop was obtained (0.4 g, 10 %, 97 % ee, S). The residue was dissolved in MeOH (0.2 ml) under reflux, the solution was then cooled to room temperature. The D-S salt seed was added, and further recrystallisation gave the fourth crop (0.2 g, 4.5 %, 99.4 % ee, S).

The first crop obtained from recrystallisation (0.26 g) was treated with 48% NaOH (4.5 ml) ice water (15 ml). A total of 15 ml MTBE was used to extract out the free methylamino intermediate completely in optically pure form (0.12 g, 100 %, S, (α)_{D} = (-)5.69 (c=1.16 abs. EtOH)).

### d) Preparation of 1-(2-bromoethyl)-3,4-dimethozybenzene

Triphenylphosphine (1.64 g, 6.2 mmol) was added to a solution of 2-(3,4-dimethoxyphenyl)-1-ethanol (1.027 g, 5.64 mmol) in dichloromethane (6 ml) at 0°C, followed by the dropwise addition of bromine (1.0 g, 6.2 mmol). The reaction was quenched with water after 2 hours. Usual work-up gave the crude product (1.468 g), which was taken directly to the next step.

### e) Preparation of 2-(3,4-Dimethoxyphenyl)-5-[2-(3,4-dimethoxyphenyl)ethyl-N-methylamino]-2-isopropyl pentanenitrile

A mixture of the optically pure methylamino intermediate from step c) above (0.203 g, 0.7 mmol), 1-(2-bromoethyl)-3,4-dimethoxybenzene (0.189 g, 0.77 mmol), and K₂CO₃ (0.293 g, 2.1 mmol) in acetonitrile (6 ml) was heated up to reflux for 3 days. The reaction mixture was then quenched with water and extracted with MTBE. The MTBE extract was acidified with 2N HC1, and further extracted with 2N HC1. The organic layer was disposed of. The combined aqueous layers were then basified with 48% NaOH, and extracted with diethyl ether. Usual work-up gave the desired product (0.30 g, 94%).

## Claims

1. A process for preparing a substantially single enantiomer (R or S) of
R'-NH-(CH₂)₃-C(Ar)(CN)-R,
the process comprising reacting together
R-CH(Ar)-CN and X-(CH₂)₃-A
to give
X-(CH₂)₃-C(Ar)(CN)-R,
reacting that with R'NH₂ to give
R'-NH-(CH₂)₃-C(Ar)(CN)-R,
and resolving that into a substantially single enantiomer thereof, wherein Ar is aryl, R and R' are each independently C₁₋₂₀ alkyl, X is a halo atom and A is a leaving group and the resolution is carried out using a chiral acid.

2. A process according to claim 1, wherein R' is methyl.

3. A process according to claim 1 or claim 2, wherein Ar is 3,4-dimethoxyphenyl and R is isopropyl.

4. A process according to any of claims 1 to 3, wherein the chiral acid is selected from di-o,o'-toluyltartaric acid, lithocholic acid and (R)-α-methoxy-2-(trifluoromethyl)phenylacetic acid.

5. A process for preparing a substantially single enantiomer (R or S) of a compound of the formula
Ar'-(CH₂)₂-NR'-(CH₂)₃-C(Ar)(CN)-R,
the process comprising carrying out the process of any preceding claim and reacting the product thereof with Ar'-(CH₂)₂-A', wherein Ar' is aryl and A' is a leaving group.

6. A process according to claim 5, wherein Ar' is 3,4-dimethoxyphenyl

7. A process for preparing a substantially single enantiomer (R or S) of verapamil, the process comprising carrying out the process of claim 3 and reacting the product thereof with Ar'-(CH₂)₂-A' as defined in claim 6.

## Patentansprüche

1. Verfahren zur Herstellung eines im wesentlichen einzelnen Enantiomers (R oder S) von
R'-NH-(CH₂)₃-C(Ar)(CN)-R,
umfassend das zusammen Umsetzen von
R-CH(Ar)-CN und X-(CH₂)₃-A
um
X-(CH₂)₃-C(Ar)(CN)-R
zu ergeben, die Umsetzung desselben mit R'NH₂, um
R'-NH-(CH₂)₃-C(Ar)(CN)-R
zu liefern, und die Spaltung desselben in ein im wesentlichen einzelnes Enantiomer desselben, worin Ar für Aryl steht, R und R' jeweils unabhängig C₁₋₂₀-Alkyl darstellen, X ein Halogenatom bedeutet und A eine Abgangsgruppe ist, wobei die Spaltung unter Verwendung einer chiralen Säure durchgeführt wird.

2. Verfahren nach Anspruch 1, in welchem R' für Methyl steht.

3. Verfahren nach Anspruch 1 oder Anspruch 2, in welchem Ar für 3,4-Dimethoxyphenyl steht und R Isopropyl bedeutet.

4. Verfahren nach irgendeinem der Ansprüche 1 bis 3, in welchem die chirale Säure aus Di-o,o'-toluylweinsäure, Lithocholinsäure und (R)-α-Methoxy-2-(trifluormethyl)phenylessigsäure ausgewählt wird.

5. Verfahren zur Herstellung eines im wesentlichen einzelnen Enantiomers (R oder S) einer Verbindung der Formel
Ar'-(CH₂)₂-NR'-(CH₂)₃-C(Ar)(CN)-R,
umfassend die Durchführung des Verfahrens nach irgendeinem vorangehenden Anspruch und die Umsetzung des Produkts desselben mit Ar'-(CH₂)₂-A', worin Ar' für Aryl steht und A' eine Abgangsgruppe bedeutet.

6. Verfahren nach Anspruch 5, in welchem Ar' 3,4-Dimethoxyphenyl ist.

7. Verfahren zur Herstellung eines im wesentlichen einzelnen Enantiomers (R oder S) von Verapamil, umfassend die Durchführung des Verfahrens nach Anspruch 3 und die Umsetzung des Produkts desselben mit Ar'-(CH₂)₂-A', wie in Anspruch 6 definiert.

## Revendications

1. Procédé de préparation d'un énantiomère (R ou S) sensiblement seul de
R'-NH-(CH₂)₃-C(Ar)(CN)-R
le procédé comprenant la mise à réagir de
R-CH(Ar)-CN et X-(CH₂)₃-A
pour obtenir
X-(CH₂)₃-C(Ar)(CN)-R,
la mise à réagir de ce produit avec R'NH₂ pour obtenir
R'-NH-(CH₂)₃-C(Ar)(CN)-R
et sa résolution en un énantiomère sensiblement seul, où Ar est un groupe aryle, R et R' sont chacun indépendamment un groupe alkyle en C₁₋₂₀, X est un atome d'halogène et A est un groupe qui part et la résolution s'effectue au moyen d'un acide chiral.

2. Procédé selon la revendication 1, où R' est un groupe méthyle.

3. Procédé selon la revendication 1 ou la revendication 2, où Ar est un groupe 3,4-diméthoxyphényle et R est un groupe isopropyle.

4. Procédé selon l'une quelconque des revendications 1 à 3, où l'acide chiral est sélectionné parmi l'acide di-o,o'-toluyltartrique, l'acide lithocholique et l'acide (R)-α-méthoxy-2-(trifluorométhyl)phénylacétique.

5. Procédé de préparation d'un énantiomère (R ou S) sensiblement seul d'un composé de formule
Ar'-(CH₂)₂-NR'-(CH₂)₃-C(Ar)(CN)-R,
le procédé comprenant la mise en oeuvre du procédé selon l'une quelconque des revendications précédentes et la mise à réagir de son produit avec Ar'-(CH₂)₂-A', où Ar' est un groupe aryle et A' est un groupe qui part.

6. Procédé selon la revendication 5, où Ar' est un groupe 3,4-diméthoxyphényle.

7. Procédé de préparation d'un énantiomère (R ou S) sensiblement seul du vérapamil, le procédé comprenant la mise en oeuvre du procédé selon la revendication 3 et la mise à réagir de son produit avec Ar'-(CH₂)₂-A' selon la revendication 6.
